# EUROPEAN PATENT SPECIFICATION

(11) **EP 3 723 778 B1**
(45) Date of publication and mention of the grant of the patent: **12.02.2025**
(21) Application number: 18816023.8
(22) Date of filing: 07.12.2018
(51) Int. Cl.: A61K 35/745, A61K 31/702, A61K 9/00, A61K 9/02, A61K 47/36, A61K 9/14, A61K 9/20, A61P 1/00

(54) **COMPOSITION FOR USE FOR TREATING DELAYED COLONIZATION BY BIFIDOBACTERIUM AFTER BIRTH**
ZUSAMMENSETZUNG ZUR VERWENDUNG ZUR BEHANDLUNG VON VERZÖGERTER KOLONISATION DURCH BIFIDOBACTERIUM NACH DER GEBURT
COMPOSITION POUR L'UTILISATION POUR TRAITER LA COLONISATION RETARDÉE PAR BIFIDOBACTERIUM APRÈS LA NAISSANCE

(30) Priority: 11.12.2017 EP 17206447
(43) Date of publication of application: 21.10.2020
(73) Proprietor: N.V. Nutricia, 2712 HM Zoetermeer (NL)
(72) Inventor: LAY, Christophe, Singapore 689676 (SG); KNOL, Jan, 3584 CT Utrecht (NL); BEN AMOR, Kaouther, 3584 CT Utrecht (NL)
(74) Representative: Nederlandsch Octrooibureau
(86) International application number: PCT/EP2018/083949
(87) International publication number: WO 2019/115382

(56) References cited:
- WO-A1-2016/100618
- US-A1- 2016 331 792

## Description

### Field of the invention

The present invention relates to therapeutic methods and compositions for therapeutic use in promoting or stimulating the development of a healthy gut microbiota in infants born with a lack of *Bifidobacterium* species at birth, such as where the mother received antibiotics during delivery (i.e. intrapartum antibiotic prophylaxis (IAP)) and particularly with infants delivered by Caesarean section.

### Background art

The worldwide rate of infant deliveries via Caesarean section has increased over the last decade, making it the most common surgical procedure performed in women of childbearing age today. While the WHO recommends that Caesarean section deliveries be indicated in 10 to 15 % of all deliveries, in many countries the rate of Caesarean section deliveries significantly exceeds this recommendation. Over the past years, the medical field has started to realise that a Caesarean section delivery introduces health risks, and the obstetrician is thus advised to assess these long- and short-term health consequences for mother and infant, as well as weigh the risks associated with the procedure itself against not performing the procedure.

In recent times the field has started to realize that infants delivered via Caesarean section suffer from the consequences of a non-existent transmission of gut microbiota from the mother to the infant at birth. EP1940250 (or US2016/100618) has clarified there is a complete lack of any detectable amounts of *Bifidobacterium* species in the gut of C-section infants compared to the presence of significant amounts of at least three different *Bifidobacterium* species of the group of *B. longum, B. breve, B. infantis, B. catenulatum, B. pseudocatenulatum, B. adolescentis, B. animalis, B. gallicum, B. lactis* and *B. bifidum* observed in infants born by natural birth. While vaginally delivered infants acquire bacterial communities from their own mother's vaginal and gut microbiota, C-section infants acquire bacterial communities from the maternal skin and the surrounding birth environment. EP1940250 (or US2016/100618) advocates the use of at least one *Bifidobacterium* species in combination with a non-digestible oligosaccharide, in order to normalize the gut microbiota in the infant. US2016/331792 describes methods and compositions for restoring normal microbiota in pre-term newborns or newborns delivered by Cesarean section and methods for preventing or ameliorating diseases associated with delivery by Cesarean section or pre-term birth comprising administering to said newborns at the time of birth or shortly thereafter an effective amount of a vaginal microbiota inoculum obtained from the newborn's mother or a donor or an effective amount of a probiotic composition.

Promoting or stimulating healthy gut microbiota in infants delivered by Caesarean section is considered a therapeutic use, given that said infants suffer from bifidobacteria deficits at birth. Compared to the normal 10⁷ - 10⁹ cfu bifidobacteria counts, an infant born by Caesarean section typically exhibits hardly any bifidobacteria at birth.

The delayed colonization by bifidobacteria lasts for longer periods, as evidenced in Figure 1A in Chua et al. "Effect of synbiotic on the gut microbiota of cesarean delivered infants: a randomized, double-blind, multicenter study" JPGN vol. 65(1) July 2017, and is believed to have an impact on all kinds of increased health risks associated with C-section such as occurrence or development of food allergy, eczema (e.g. atopic dermatitis), asthma, allergic rhinitis and/or allergic conjunctivitis.The delay in colonization by bifidobacteria can be minimized by administering to said C-section infants infant formula supplemented with synbiotic compositions comprising *B. breve* and non-digestible oligosaccharides (scGOS/IcFOS), thus restoring bifidobacteria levels within the first days of life (see Figure 1A in Chua *et al*.). Analysis by quantitative real-time polymerase chain reaction (qPCR) revealed that the synbiotic group showed a higher absolute gene count of bifidobacteria from the first days of intervention (p<0.0001) compared to the control group, and the effect remained significant until week 12 (p=0.032).

### Summary of the invention

It was found by the inventors that infants born by C-section that were either exclusively breastfed, or mixed fed, meaning breastfed and supplemented with an infant formula with or without added prebiotic, still exhibited a delayed colonization by *Bifidobacterium* species in the gut.

On the contrary, those infants born by C-section that were mixed fed with an infant formula supplemented with a synbiotic composition did not exhibit a delayed colonization by *Bifidobacterium,* or to a lesser extent. From this it was concluded that breastmilk did not redress the bifidobacteria deficiency, while intervention with synbiotics did.

More particularly, the inventors found that chronic administration of the synbiotic composition was not necessarily needed, and that bifidobacteria could be restored using *ad hoc* administration of the synbiotic composition of the invention.

Reference is made to example 3 as described herein. It is clear from Table 1D in example 3 that two shots in the first week after birth of *B*. *breve* M-16V and indigestible oligosaccharides are sufficient to initiate and maintain indigenous growth of bifidobacteria and to stimulate growth of indigenous *B*. *breve,* i.e. *B. breve* other than the strain administered. The support of indigenous bifidobacteria growth lasted for at least 22 weeks, in spite of the observation that the *Bifidobacterium* strain originally administered dropped rapidly after administration, and no further Bifidobacteria were administered after the above initial period of administration immediately after birth.

The inventors have thus found that the lack of bifidobacteria at birth can be tackled even faster if an infant born with a lack of *Bifidobacterium* species at birth is administered a composition comprising between 10³ and 10¹⁶ colony forming units (cfu) per serving of *Bifidobacterium breve,* preferably *B*. *breve* M-16V, and between 0.1-5 gram per serving of at least one indigestible oligosaccharide, wherein said non-digestible oligosaccharide comprises galactooligosaccharides (GOS) immediately after birth, i.e. once or twice within one week after birth, preferably once or twice within 5 days after birth, most preferably twice within 3 days after birth. That is, once or twice only, and within the first week after birth only, preferably within 5 days from birth, most preferably within 3 days from birth only. This is particularly suited for infants whose mothers received antibiotics during delivery and/or in infants delivered via C-section, most preferably in infants delivered via C-section.

Conveniently, there is no need to interfere with breastfeeding. Chronic administration of the synbiotic composition may thus conveniently be avoided and is preferably disclaimed. The acute treatment results in optimum effects, and bifidobacteria levels are restored to levels beyond 1*10⁷ within 5 days from birth (Table 1D).

The administration of said composition is limited to once or twice within one week after birth only, preferably once or twice within 5 days after birth only, most preferably twice within 3 days after birth only. With the term 'only' it is intended that the synbiotic composition is preferably administered in said period exclusively. Surprisingly, the incidental and limited boost with *B. breve* and indigestible oligosaccharides promotes the endogenous growth of *B*. *breve* species, in infants born via caesarean section otherwise lacking bifidobacteria at birth, and all well within the first week, in fact even after 5 days after birth.

Based on these findings, the inventors realized a simplified way of redressing the impaired gut bifidobacteria in caesarean section delivered infants, regardless the mode of feeding, preferably in breastfed caesarean section delivered infants, by minimizing the administration with synbiotics to once or twice immediately after birth.

In a preferred embodiment, the C-section infant receives breastfeeding, which is still the golden standard and is known to further optimize the gut microbiota once the initial C-section associated delayed bifidobacteria colonization is solved.

The inventors believe that administration with one or two dosages of the synbiotic early in life also works advantageously for vaginally delivered infants lacking bifidobacteria at birth to an extent similar as the delayed levels reported for C-section delivered infants, for instance where the mother received antibiotics during delivery (Intrapartum Antibiotic Prophylaxis/ IAP). In one embodiment, the infant born with a lack of *Bifidobacterium* species at birth is an infant from a mother receiving IAP.

### Description of embodiments

The inventors have found a method for therapeutically improving or stimulating the development of a healthy gut microbiota, by stimulating *Bifidobacterium* intra-species growth in infants born with a lack of *Bifidobacterium* species at birth, including infants where the mother received antibiotics during delivery (IAP), and particularly in the case of infants delivered via C-section, said method involving administering said infant, particularly an infant delivered via C-section, once or twice only a composition comprising between 10³ and 10¹⁶ colony forming units (cfu) per serving of *Bifidobacterium breve,* preferably a *Bifidobacterium breve* M-16V strain, and between 0.1-5 gram per serving of at least one non-digestible oligosaccharide, wherein said non-digestible oligosaccharide comprises galactooligosaccharides, more preferably therapeutically effective amounts of non-digestible galactooligosaccharides and fructans, wherein the composition is only administered to the infant once or twice, preferably an infant delivered via C-section, within the first week after birth only, preferably within 5 days, most preferably within 3 days from birth.

It is of utmost importance to improve and/or accelerate the development of the appropriate bifidobacteria population and *Bifidobacterium* species diversity in the gastrointestinal tract of said infants at the onset of life outside the womb. Without intervention, the bifidobacteria microbiota levels stay behind by a factor 1000 for C-section delivered infants compared to their normal delivery counterparts for a period of many weeks.

The invention pertains to a composition for therapeutic use in improving or stimulating the development of a healthy gut microbiota, by stimulating *Bifidobacterium* intra-species growth, in infants born with a lack of *Bifidobacterium* species at birth, preferably in infants whose mothers received antibiotics during delivery and/or in infants delivered via C-section, preferably in infants delivered via C-section, involving administering to the infant a composition comprising between 10³ and 10¹⁶ colony forming units (cfu) per serving of *Bifidobacterium breve* and between 0.1-5 gram per servingof at least one non-digestible oligosaccharide, wherein said non-digestible oligosaccharide comprises galactooligosaccharides, wherein the composition is administered to the infant once or twice only, and within the first week after birth only, preferably within 5 days from birth, most preferably within 3 days from birth only.

The composition is fed to said infant in the first week exclusively, preferably within 5 days from birth only, most preferably within 3 days from birth only.

In a preferred embodiment, the use involves stimulating the growth of indigenous *B*. *breve* species other than the *B*. *breve* M-16V strain administered.

Within the context of the invention, the composition comprising between 10³ and 10¹⁶ colony forming units (cfu) per serving of *Bifidobacterium breve,* preferably a *Bifidobacterium breve* M-16V strain, and between 0.1-5 gram per serving of at least one non-digestible oligosaccharide, wherein said non-digestible oligosaccharide comprises galactooligosaccharides,, more preferably therapeutically effective amounts of non-digestible galactooligosaccharides and fructans, may be referred to as the 'synbiotic composition'.

The composition according to one embodiment of the invention is for promoting indigenous bifidobacteria growth in the gut of an infant delivered via Caesarean section (C-section). In a preferred method according to the invention, the composition according to the invention is administered to an infant delivered via Caesarean section. Caesarean section is a surgical procedure where an infant is delivered through an incision made in the mother's abdominal wall, and then through the wall of the uterus. A Caesarean section is typically performed when it is safer for the mother or the infant than a vaginal delivery, or in case the mother prefers to have a caesarean section rather than deliver her infant vaginally.

Infants born via Caesarean section are known to have an impaired or delayed gastrointestinal *Bifidobacterium* population, which is attributed to the mode of delivery. With an impaired or delayed bifidobacteria population it is generally understood total bifidobacteria levels are less than 1*10⁶ cfu, preferably less than 1*10⁵ cfu.

The consequences of improving the gastrointestinal *Bifidobacterium* population according to the invention are a reduced risk of occurrence or development of food allergy, eczema (e.g. atopic dermatitis), asthma, allergic rhinitis and/or allergic conjunctivitis in infants delivered by C-section.

Improvement of the gastrointestinal *Bifidobacterium* population in terms of numbers and diversity of species may also result in a reduced risk of infections, including gastrointestinal infections, and reducing the occurrence and/or severity of infections including gastrointestinal infections.

C-section has also been associated with increased risks of developing allergies and obesity later in life, and the improvements in terms of swift microbiota restoration achieved by the invention thus also relate to reducing the risks of developing allergies and obesity later in life.

The composition according to the invention is typically suitable for enteral administration to the infant. The composition may be provided in any form known in the art to be suitable for such administration, such as in solid form, in semi-solid form or in liquid form. Preferably, the composition is a nutritional composition or a nutritional supplement. The composition may be referred to as a nutritional composition, preferably a nutritional composition for providing nutrition to infants, in particular infants delivered via Caesarean section. Preferably, the composition is in the form of a liquid or in the form of a powder supplement, which can be reconstituted with a liquid (typically water) to obtain a liquid composition. Alternatively, the composition is in the form of a capsule or tablet. In one embodiment, the composition is not an infant formula.

The composition according to the invention is preferably a powder supplement, wherein the powder supplement weighs between 0.1-20 gram per serving, more preferably between 0.2-10 gram per serving and most preferably between 0.5-5 gram per serving.

The composition according to the invention is preferably a suppository, pill or tablet, wherein the suppository, pill or tablet weighs between 0.1-10 gram per serving, more preferably between 0.2-5 gram per serving and most preferably 0.5-2.5 gram per serving.

The *B*. *breve* strain is provided in therapeutically effective amounts. The present composition preferably contains between 10³ and 10¹³ colony forming units (cfu) *B*. *breve* per gram dry weight of the present composition, preferably between 10⁴ and 10¹², more preferably between 10⁵ and 10¹⁰. Preferably, the present composition contains between 10³ and 10¹³ colony forming units (cfu) bifidobacteria per g dry weight of the present composition, more preferably between 10⁴ and 10¹², most preferably between 10⁵ and 10¹².

In terms of doses, the present composition provides between 10³ and 10¹⁶ cfu, more preferably between 10⁴ and 10¹⁵ cfu, most preferably between 10⁵ and 10¹² cfu *B*. *breve* per serving. Preferably, the present composition provides between 10³ and 10¹⁶ cfu, more preferably between 10⁴ and 10¹⁵ cfu, most preferably between 10⁵ and 10¹² cfu bifidobacteria per serving. An especially suitable strain of *B*. *breve* to be used in the present invention is *B*. *breve* M-16V.

In one embodiment, the composition does not comprise any *Bifidobacterium* species other than *B. breve* M-16V.

In one embodiment, stimulating the development of a healthy gut microbiota involves stimulating *Bifidobacterium* intra-species growth other than the *Bifidobacterium* species part of the synbiotic composition of the invention.

Preferably, the composition comprises *B*. *breve* in freeze-dried form, which is especially suitable when the composition is in powder, capsule or tablet form.

In one embodiment, the composition according to the invention comprises a mixture of galacto-oligosaccharides and fructans.

Galacto-oligosaccharides and fructans are non-digestible oligosaccharides, also referred to as a "prebiotic" or "prebiotic fibre". In the context of the present invention, the term "non-digestible oligosaccharide" refers to oligosaccharides which are not digested in the intestine by the action of digestive enzymes present in the upper digestive tract (small intestine and stomach) of the infant but instead are fermented by the intestinal microbiota of said infant, thus conferring benefits upon the host wellbeing and health.

Preferably the present non-digestible oligosaccharides have a degree of polymerisation (DP) of 2 to 250, preferably an average DP 2 to 100, more preferably 2 to 60. Preferably, at least 50 wt.% of the present non-digestible oligosaccharides have an average degree of polymerisation in the range of 2 to 60.

The terms "prebiotic" and "prebiotic fibre" refer to non-digestible fibres that beneficially affects the host by selectively stimulating the growth and/or activity of one or a limited number of bacterial species in the colon. Health effects of prebiotics, also in combination with probiotics, are described in Collins, Am. J. Clin. Nutr. 1999, 69(suppl.), 10525-10575.

Preferably, the present non-digestible oligosaccharide is soluble. The term "soluble" as used herein, when having reference to a fibre or oligosaccharide, means that the substance is at least 50% soluble according to the method described by Prosky et al. in J. Assoc. Off. Anal. Chem. 1988, 71, 1017-1023.

The non-digestible oligosaccharides of the invention comprise galacto-oligosaccharides, in particular beta-galacto-oligosaccharides. Herein, galactose units make up for at least 50% of the monosaccharide units. The galacto-oligosaccharides are preferably [galactose]ₙ-glucose; wherein n is an integer between 1 and 60, i.e. 2, 3, 4, 5, 6,...., 59, 60; preferably n is 2, 3, 4, 5, 6, 7, 8, 9 and/or 10, a good example being trans-galacto-oligosaccharides. The galactose units are preferably beta-linked. The galacto-oligosaccharides preferably comprise saccharides with an average degree of polymerisation (DP) of 2 to 10 (scGOS). (Trans)galactooligosaccharide is for example available under the trade name Vivinal^{®}GOS (Borculo Domo Ingredients, Zwolle, Netherlands), Bimuno (Clasado), Cup-oligo (Nissin Sugar) and Oligomate55 (Yakult).

Preferably, the non-digestible oligosaccharides may further comprise fructans. Preferred fructans include fructo-oligosaccharides, fructo-polysaccharides, inulin and mixtures thereof, most preferably fructo-oligosaccharides. Preferred fructo-oligosaccharides are short-chain fructo-oligosaccharides (scFOS), having an average DP in the range of 2 to 10, and long-chain fructo-oligosaccharide (IcFOS), having an average DP in the range of 10 to 60, preferably in the range of 15 - 40. scFOS is commercially available as Beneo^{®} P95 or Raftilose P95 (Orafti). A particular type of IcFOS is inulin, such as Raftilin HP.

In an especially preferred embodiment, the present composition comprises galacto-oligosaccharides and fructo-oligosaccharides and/or fructo-polysaccharides, most preferably scGOS and IcFOS (preferably said IcFOS having an average DP > 15). In the present embodiment, galacto-oligosaccharides and fructans are preferably present in a weight ratio 5:1 - 20:1, even more preferably 7:1 - 15:1, even more preferably 8:1 - 10:1, most preferably about 9:1.

The non-digestible oligosaccharides are present in therapeutically effective amounts. The present composition preferably comprises 0.05 to 50 wt% of said non-digestible oligosaccharides, more preferably 0.5 to 25 wt%, even more preferably 1 to 15 wt%, most preferably 2 to 10 wt%, based on dry weight of the composition. In terms of doses, the present composition provides between 0.1 and 5 gram non-digestible oligosaccharides per serving.

Administration of the present composition to the infant preferably occurs by administering to the infant directly. Administration typically occurs enterally, i.e. directly into the gastrointestinal tract of the infant. Enteral administration includes oral administration, tube feeding, stoma feeding and rectal administration. Oral administration is preferred.

The composition may be in any form known in the art to be suitable for enteral administration, such as in solid form, in semi-solid form or in liquid form. The composition is preferably a powder suited to be reconstituted with water, a nutritional supplement, a suppository, a pill or a tablet. Wherever doses per serving are defined above for the composition according to the invention, the method according to the inventions preferably involves administration of a serving containing said dose.

In one embodiment, the composition is administered to the infant, provided in the form of
(i) a liquid having a volume between 0.5 to 5 ml for oral administration, wherein said liquid is preferably administered to said infant with a syringe, pipette or tube; or
(ii) a suppository, pill or tablet, and wherein said composition is administered to the infant rectally.

In one embodiment, the composition may be provided in the form of a unit dose form, which refers to individual single-use packages. The composition may be present in a container containing one single or more unit dose(s). Preferably, the present composition is accompanied with instructions for use.

### Examples

### Example 1

A single use sachet comprising about 2 grams of a powdered composition for providing one serving. The powdered composition is suitable for addition to infant formula or breast milk in the first week after birth.

The serving comprises 1.0 g of scGOS/IcFOS (about 9:1 weight ratio), 9 x 10^8 cfu *B*. *breve* M-16V and soluble digestible carbohydrates. 'scGOS' were short-chain galactooligosaccharides (Vivinal-GOS^{™}; Borculo Domo Ingredients, Netherlands; Degree of Polymerisation [DP] 2 - 8), 'IcFOS' were long-chain fructooligosaccharides (Raftiline HP^{™}, Orafti, Tienen, Belgium; average DP 22 - 25).

### Example 2

A total of 183 infants born to healthy pregnant mothers were enrolled in a clinical study. Eligible participants included healthy term neonates born by elective C-section. In the study, infants born by C-section were randomized to receive a control formula (n=50), or the same formula containing synbiotics (scGOS/IcFOS and *Bifidobacterium breve* M-16V, n=52), or prebiotics (scGOS/IcFOS, n=51) immediately after birth, for a period of 16 weeks, and a 6 weeks follow-up. Thirty subjects born vaginally were included as a non-randomized reference group.

Fecal samples were collected during the study to determine the efficacy of the nutritional intervention. The composition of the gut microbiota was determined through qPCR targeting total bacteria, total *Bifidobacterium* species, total *B*. *breve* species and the probiotic strain *B*. *breve* M-16V. The limit of detection in this assay was around 4.6 log 10 copies/ g of feces, meaning that if for example no *B*. *breve* is present in the sample, then the qPCR measurement for *B*. *breve* will be 4.6 log 10 copies/ g of feces.

### Example 3

The gut microbiota distributions that are described in Table's 1A-1D are taken from the clinical study, which is described in Example 2. Table's 1A-1D describe the levels of bacteria determined by qPCR in the stool samples of four individual subjects.

Table 1A (Subject 77) and 1B (Subject 150) show the gut microbiota distribution of two individual infants born via C-section who received breastfeeding from birth until day 2, and a mix of formula feeding (without synbiotics) and breastfeeding onwards. These 'control' infants did not receive the composition of the invention. For Table 1A, no bifidobacteria were detected at day 3; data are missing at day 5 and at week 2; for Table 1B, no bifidobacteria were detected at day 3, day 5 and week 2; data are missing at week 12. Both infants show increased bifidobacteria levels at week 4. Breast-milk was not found to redress bifidobacteria levels in C-section born infants 77 and 150 in the first weeks of life.

Table 1C (subject 74) shows the gut microbiota distribution of an individual infant born via C-section who received breastfeeding until day 5, followed by mixed feeding including synbiotic formula with *B. breve* M-16V and scGOS/IcFOS. No *Bifidobacterium* species and *B. breve* M-16V were detected at day 5; normal bifidobacteria levels were found at week 2. Data are missing for day 3.

Table 1D (subject 109) shows the gut microbiota distribution of an individual infant born via C-section who received breastfeeding, but also one shot of a formula (85ml, 0.7 g of scGOS/IcFOS, 6.4 × 10⁸ cfu *B. breve* M-16V) at birth and another at day 2 (65ml, 0.5 g of scGOS/IcFOS, 4.9 × 10⁸ cfu *B*. *breve* M-16V). Data are missing at day 3 and week 2. While *B*. *breve* M-16V levels drop rapidly after the two shots of the intervention formula, total bifidobacteria including *B*. *breve* show normal levels already at day 5. It evidences that the synbiotic shots according to the invention stimulate indigenous growth, at the onset. These normal bifidogenic levels are maintained even after 22 weeks. Since the level of *B*. *breve* species was maintained across the whole study period, those data indicate that a one/two shot supplementation of synbiotic in the first days of life promoted intra-species diversity of *Bifidobacterium breve* that was sustained by breastfeeding, and already after 5 days.

**Table 1A - Subject no. 77**

| **Count (log 10 copies / g of feaces)** | **Day 3** | **Day 5** | **Wk 02** | **Wk 04** | **Wk 08** | **Wk 12** | **Wk 16** | **Wk 22** |
|---|---|---|---|---|---|---|---|---|
| *B. breve* M-16V | 4.6 | - | - | 4.6 | 4.6 | 4.6 | 4.6 | 4.6 |
| *B. breve* | 4.7 | - | - | 8.38 | 8.47 | 7.78 | 7.84 | 7.29 |
| Total bifidobacteria | 4.6 | - | - | 8.52 | 8.29 | 7.58 | 7.86 | 7.10 |
| Total bacteria | 8.65 | - | - | 9.16 | 9.08 | 8.27 | 8.61 | 7.78 |

**Table 1B - Subject no. 150**

| **Count (log 10 copies/ g of feaces)** | **Day 3** | **Day 5** | **Wk 02** | **Wk 04** | **Wk 08** | **Wk 12** | **Wk 16** | **Wk 22** |
|---|---|---|---|---|---|---|---|---|
| *B. breve* M-16V | 4.6 | 4.6 | 4.6 | 4.6 | 4.6 | - | 4.6 | 4.6 |
| *B. breve* | 4.7 | 4.7 | 4.7 | 4.7 | 9.12 | - | 8.74 | 8.37 |
| Total bifidobacteria | 4.6 | 4.6 | 4.6 | 7.76 | 8.21 | - | 7.69 | 8.05 |
| Total bacteria | 7.12 | 8.52 | 8.48 | 8.23 | 8.72 | - | 8.89 | 9.55 |

**Table 1C - Subject no. 74**

| **Count (log 10 copies/ g of feaces)** | **Day 3** | **Day 5** | **Wk 02** | **Wk 04** | **Wk 08** | **Wk 12** | **Wk 16** | **Wk 22** |
|---|---|---|---|---|---|---|---|---|
| *B. breve* M-16V | - | 4.6 | 7.44 | 7.35 | 7.25 | 8.72 | 9.17 | 6.84 |
| *B. breve* | - | 4.7 | 7.86 | 7.67 | 7.58 | 8.92 | 9.32 | 7.05 |
| Total bifidobacteria | - | 4.6 | 7.55 | 7.8 | 7.42 | 9.16 | 9.16 | 7.48 |
| Total bacteria | - | 6.88 | 8.49 | 8.80 | 8.89 | 9.53 | 9.63 | 7.90 |

**Table 1D - Subject no. 109**

| **Count (log 10 copies/ g of feaces)** | **Day 3** | **Day 5** | **Wk 02** | **Wk 04** | **Wk 08** | **Wk 12** | **Wk 16** | **Wk 22** |
|---|---|---|---|---|---|---|---|---|
| *B. breve* M-16V | - | 7.69 | - | 4.6 | 4.6 | 4.6 | 4.6 | 4.6 |
| *B. breve* | - | 7.81 | - | 8.04 | 7.46 | 7.57 | 8.03 | 7.99 |
| Total bifidobacteria | - | 7.70 | - | 7.95 | 7.55 | 7.47 | 8.01 | 7.79 |
| Total bacteria | - | 8.18 | - | 8.78 | 8.97 | 8.40 | 8.67 | 8.67 |

## Claims

1. A composition for therapeutic use in improving or stimulating the development of a healthy gut microbiota, by stimulating *Bifidobacterium* intra-species growth, in infants born with a lack of *Bifidobacterium* species at birth, preferably in infants whose mothers received antibiotics during delivery and/or in infants delivered via C-section, preferably in infants delivered via C-section, involving administering to the infant a composition comprising between 10³ and 10¹⁶ colony forming units (cfu) per serving of *Bifidobacterium breve* and between 0.1-5 gram per serving of at least one non-digestible oligosaccharide, wherein said non-digestible oligosaccharide comprises galactooligosaccharides, wherein the composition is administered to the infant once or twice only, and within the first week after birth only.

2. The composition for use according to claim 1, wherein said *Bifidobacterium breve* is *Bifidobacterium breve* M-16V.

3. The composition for use according to any one of the preceding claims, wherein said non-digestible oligosaccharide comprises short-chain galacto-oligosaccharides with an average degree of polymerisation (DP) of 2 to 10.

4. The composition for use according to any one of the preceding claims, wherein said non-digestible oligosaccharide comprises galactooligosaccharides and fructans.

5. The composition for use according to claim 4, wherein the fructans include fructo-oligosaccharides, fructo-polysaccharides, inulin and mixtures thereof, preferably short-chain fructo-oligosaccharides (scFOS) having an average DP in the range of 2 to 10 and/or long-chain fructo-oligosaccharide (IcFOS) having an average DP in the range of 10 to 60.

6. The composition for use according to any one of the preceding claims, wherein said composition is administered within 5 days from birth.

7. The composition for use according to any one of the preceding claims, wherein said infant is breastfed.

8. The composition for use according to any one of the preceding claims, stimulating *Bifidobacterium* intra-species growth other than the *Bifidobacterium breve* administered.

9. The composition for use according to any one of the preceding claims, wherein the composition is administered to the infant within 5 days from birth exclusively.

10. The composition for use according to any one of the preceding claims, wherein the composition is a powder supplement, a suppository, pill or tablet.

11. The composition for use according to claim 10, wherein the composition is a powder supplement, wherein the powder supplement weighs between 0.1-20 gram per serving; or wherein the composition is a suppository, pill or tablet, wherein the suppository, pill or tablet weighs between 0.1-10 gram per serving.

## Patentansprüche

1. Zusammensetzung zur therapeutischen Anwendung bei der Verbesserung oder Stimulierung der Entwicklung einer gesunden Darmmikrobiota durch Stimulieren des intra-species-Wachstums von *Bifidobacterium* bei Säuglingen, die mit einem Mangel an *Bifidobacterium*-Spezies bei der Geburt geboren wurden, vorzugsweise bei Säuglingen, deren Mütter während der Geburt Antibiotika erhalten haben, und/oder bei Säuglingen, die per Kaiserschnitt entbunden wurden, vorzugsweise bei Säuglingen, die per Kaiserschnitt entbunden wurden, wobei dem Säugling eine Zusammensetzung verabreicht wird, die zwischen 10³ und 10¹⁶ koloniebildende Einheiten (kbE) pro Portion von *Bifidobacterium breve* und zwischen 0,1-5 Gramm pro Portion mindestens eines nicht verdaulichen Oligosaccharids umfasst, wobei das nicht verdauliche Oligosaccharid Galactooligosaccharide umfasst, wobei die Zusammensetzung dem Säugling nur einmal oder zweimal und nur innerhalb der ersten Woche nach der Geburt verabreicht wird.

2. Zusammensetzung zur Anwendung nach Anspruch 1, wobei das *Bifidobacterium breve Bifidobacterium breve* M-16V ist.

3. Zusammensetzung zur Anwendung nach einem der voranstehenden Ansprüche, wobei das nicht verdauliche Oligosaccharid kurzkettige Galacto-Oligosaccharide mit einem durchschnittlichen Polymerisationsgrad (DP) von 2 bis 10 umfasst.

4. Zusammensetzung zur Anwendung nach einem der voranstehenden Ansprüche, wobei das nicht verdauliche Oligosaccharid Galaktooligosaccharide und Fruktane umfasst.

5. Zusammensetzung zur Anwendung nach Anspruch 4, wobei die Fruktane Fructo-Oligosaccharide, Fructo-Polysaccharide, Inulin und Mischungen davon einschließen, vorzugsweise kurzkettige Fructo-Oligosaccharide (scFOS) mit einem durchschnittlichen DP im Bereich von 2 bis 10 und/oder langkettige Fructo-Oligosaccharide (IcFOS) mit einem durchschnittlichen DP im Bereich von 10 bis 60.

6. Zusammensetzung zur Anwendung nach einem der voranstehenden Ansprüche, wobei die Zusammensetzung innerhalb von 5 Tagen nach der Geburt verabreicht wird.

7. Zusammensetzung zur Anwendung nach einem der voranstehenden Ansprüche, wobei der Säugling gestillt wird.

8. Zusammensetzung zur Anwendung nach einem der voranstehenden Ansprüche, die das Wachstum von *Bifidobacterium* intra-species stimuliert, das nicht das verabreichte *Bifidobacterium breve* ist.

9. Zusammensetzung zur Anwendung nach einem der voranstehenden Ansprüche, wobei die Zusammensetzung dem Säugling ausschließlich innerhalb von 5 Tagen nach der Geburt verabreicht wird.

10. Zusammensetzung zur Anwendung nach einem der voranstehenden Ansprüche, wobei die Zusammensetzung ein pulverförmiges Ergänzungsmittel, ein Zäpfchen, eine Pille oder eine Tablette ist.

11. Zusammensetzung zur Verwendung nach Anspruch 10, wobei die Zusammensetzung ein pulverförmiges Ergänzungsmittel ist, wobei das pulverförmige Ergänzungsmittel zwischen 0,1-20 Gramm pro Portion wiegt; oder wobei die Zusammensetzung ein Zäpfchen, eine Pille oder Tablette ist, wobei das Zäpfchen, die Pille oder Tablette zwischen 0,1-10 Gramm pro Portion wiegt.

## Revendications

1. Composition pour utilisation thérapeutique afin d'améliorer ou stimuler le développement d'un microbiote intestinal sain, en stimulant la croissance intra-espèce de *Bifidobacterium,* chez des nourrissons nés avec un manque d'espèces de *Bifidobacterium* à la naissance, de préférence chez des nourrissons dont les mères ont reçu des antibiotiques pendant l'accouchement et/ou chez les nourrissons accouchés par césarienne, de préférence chez des nourrissons accouchés par césarienne, impliquant l'administration au nourrisson d'une composition comprenant entre 10³ et 10¹⁶ unités formant colonie (ufc) par portion de *Bifidobacterium breve* et entre 0,1 et 5 grammes par portion d'au moins un oligosaccharide non digestible, dans laquelle ledit oligosaccharide non digestible comprend des galactooligosaccharides, dans laquelle la composition est administrée au nourrisson uniquement une ou deux fois, et uniquement dans la première semaine après la naissance.

2. Composition pour utilisation selon la revendication 1, dans laquelle ladite *Bifidobacterium breve* est la *Bifidobacterium breve* M-16V.

3. Composition pour utilisation selon l'une quelconque des revendications précédentes, dans laquelle ledit oligosaccharide non digestible comprend des galactooligosaccharides à chaîne courte avec un degré moyen de polymérisation (DP) de 2 à 10.

4. Composition pour utilisation selon l'une quelconque des revendications précédentes, dans laquelle ledit oligosaccharide non digestible comprend des galactooligosaccharides et des fructanes.

5. Composition pour utilisation selon la revendication 4, dans laquelle les fructanes comprennent des fructooligosaccharides, des fructopolysaccharides, de l'inuline et des mélanges de ceux-ci, de préférence des fructooligosaccharides à chaîne courte (scFOS) ayant un DP moyen dans la plage de 2 à 10 et/ou un fructooligosaccharide à chaîne longue (IcFOS) ayant un DP moyen dans la plage de 10 à 60.

6. Composition pour utilisation selon l'une quelconque des revendications précédentes, dans laquelle ladite composition est administrée dans les 5 jours après la naissance.

7. Composition pour utilisation selon l'une quelconque des revendications précédentes, dans laquelle le nourrisson est allaité.

8. Composition pour utilisation selon l'une quelconque des revendications précédentes, stimulant la croissance intra-espèce de *Bifidobacterium* autre que la *Bifidobacterium breve* administrée.

9. Composition pour utilisation selon l'une quelconque des revendications précédentes, dans laquelle la composition est administrée au nourrisson exclusivement dans les 5 jours après la naissance.

10. Composition pour utilisation selon l'une quelconque des revendications précédentes, dans laquelle la composition est un complément en poudre, un suppositoire, une pilule ou un comprimé.

11. Composition pour utilisation selon la revendication 10, dans laquelle la composition est un complément en poudre, dans laquelle le complément en poudre pèse entre 0,1 et 20 grammes par portion ; ou dans laquelle la composition est un suppositoire, une pilule ou un comprimé, dans laquelle le suppositoire, la pilule ou le comprimé pèse entre 0,1 et 10 grammes par portion.
